# EUROPEAN PATENT SPECIFICATION

(11) **EP 0 931 093 B1**
(45) Date of publication and mention of the grant of the patent: **16.04.2008**
(21) Application number: 97940148.6
(22) Date of filing: 01.09.1997
(51) Int. Cl.: C07K 14/205, C07K 16/12, C12N 15/10, C12N 15/31, C12N 15/74, A61K 39/112

(54) **HELICOBACTER PYLORI LIVE VACCINE**
LEBEND-IMPFSTOFF GEGEN HELIOBACTER PYLORI
VACCIN VIVANT CONTRE HELICOBACTER PYLORI

(30) Priority: 11.10.1996 EP 96116337
(43) Date of publication of application: 28.07.1999
(73) Proprietor: Max-Planck-Gesellschaft zur Förderung der Wissenschaften e.V., 80539 München (DE)
(72) Inventor: MEYER, Thomas, F., D-72076 Tübingen (DE); HAAS, Rainer, D-81547 München (DE); YAN, Zhengxin, D-72076 Tübingen (DE); GOMEZ-DUARTE, Oscar, Baltimore, MD 21212 (US); LUCAS, Bernadette, D-12167 Berlin (DE)
(74) Representative: Weiss, Wolfgang
(86) International application number: PCT/EP1997/004744
(87) International publication number: WO 1998/016552

(56) References cited:
- WO-A-92/11027
- WO-A-93/07273
- WO-A-93/10214
- WO-A-94/26901
- WO-A-95/02048
- WO-A-96/26740
- A.A. LINDBERG : "The history of live bacterial vaccines" DEVELOPMENTS IN BIOLOGICAL STANDARDIZATION, vol. 84, 1995, BASEL CH, pages 211-219, XP002051591

## Description

The present invention relates to novel recombinant live vaccines, which provide protective immunity against an infection by Helicobacter pylori and a method of screening H. pylori antigens for optimized vaccines.

Helicobacter is a gram-negative bacterial pathogen associated with the development of gastritis, pepic ulceration and gastric carcinoma. Several Helicobacter species colonize the stomach, most notably H. pylori, H. heilmanii and H. felis. Although H. pylori is the species most commonly associated with human infection, H. heilmanii and H. felis also have been found to infect humans. High H. pylori infection rates are observed in third world countries, as well as in industrialized countries. Among all the virulence factors described in H. pylori, urease is known to be essential for colonisation of gnobiotic pigs and nude mice. Urease is an enzyme composed of two structural subunits (UreA and UreB). Previous studies have indicated that oral immunization using recombinant UreB plus cholera toxin were able to protect mice from gastric colonisation with H. felis and H. pylori (Michetti et al., Gastroenterology 107 (1994), 1002-1011). By oral administration of recombinant UreB antigens, however, in several cases only an incomplete protection can be obtained. Other H. pylori antigens shown to give partial protection are the 87 kD vacuolar cytotoxin VacA (Cover and Blaser, J. Biol. Chem. 267 (1992), 10570; Marchetti et al., Science 267 (1995), 1655) and the 13 and 58 kD heat shock proteins HspA and HspB (Ferrero et al., Proc. Natl. Acad. Sci. USA 92 (1995), 6499).

Attenuated pathogens, e.g. bacteria, such as Salmonella, are known to be efficient live vaccines. The first indications of the efficacy of attenuated Salmonella as good vaccine in humans came from studies using a chemically mutagenized Salmonella typhi Ty21a strain (Germanier and Furer, J. Infect. Dis. 141 (1975), 553-558), tested successfully in adult volunteers (Gilman et al., J. Infect. Dis. 136 (1977), 717-723) and later on in children in a large field trial in Egypt (Whadan et al., J. Infect. Dis. 145 (1982), 292-295). The orally administered Ty21a vaccine was able to protect 96% of the Egyptian children vaccinated during three years of surveillance. Since that time new attenuated Salomonella live vector vaccines have developed (Hone et al., Vaccine 9 (1991), 810-816), in which well defined mutations incorporated into the chromosome gave rise to non-virulent strains able to induce strong immune responses after oral administration (Tacket et al., Vaccine 10 (1992), 443-446 and Tacket et al., Infect. Immun. 60 (1992), 536-541). Other advantages of the live attenuated Salmonella vaccine include its safety, easy administration, long-time protection and no adverse reactions in comparison with the former inactivated wholesale typhoid vaccines (Levine et al., Typhoid Fever Vaccines. In: Plotkin S.A., Mortimer E.A. Jr. (eds.) Vaccines. Philadelphia: WB Saunders (1988), 333-361).

Mutants of S. typhimurium have been extensively used to deliver antigens because of the possibility to use mice as an animal model, which is believed to mimick S. typhi infections in humans. The attenuation of S. typhimurium most commonly used consists in site directed mutagenesis of genes affecting the synthesis of aromatic amino acids. Such strains, designated aro mutants, have a negligible pathogenicity, as demonstrated in animal models and human trials using these constructs (Hoiseth and Stocker, Nature 291 (1981), 238-239; Tacket et al. (1992), Supra). Advantage has been taken from the potent immunogenicity of live Salmonella vaccine to deliver heterologous antigens. Expression of specific antigens in attenuated Salmonella has conferred murine protection against several bacterial pathogens.

Lindberg et al. (Dev. Biol Stand., 1995, 84:211-219) review the history of live bacterial vaccines.

WO95/02048 relates to a Salmonella cell attenuated by a first mutation in the PhoP regulon and a second mutation in an aromatic amino acid synthetic gene, or by mutation in one or more PhoP-activated or one or more PhoP-repressed genes.

Ferrero et al. (Gut 1995, 37:A51, abstract 203) relates to protection of mice by immunisation with H. pylori HspA and UreB against H. felis infection.

Hopkins et al. (Infection and Immunity, 1995, 63:3279-3286) relates to a recombinant Salmonella typhimurium vaccine encoding a hybrid form of the hepatitis B virus core antigen. This vaccine induces local immunity upon mucosal immunisation.

Kreiss et al. (Lancet 1996, 347:1630-1631) demonstrated safety of oral immunisation with recombinant urease in patients with Helicobacter pylori infection.

The use of recombinant live vaccines, which are capable of expressing Helicobacter antigens and protecting the vaccinated animals, has not yet been described. The use of attenuated live vaccines for the treatment of a Helicobacter infection has also not been rendered obvious. The reason therefor being that in the course of the Helicobacter infection a strong immune response against the pathogen per se is induced, which, however, does not lead to a protective immunity. Thus, it was highly surprising that a protective immune response is achieved when using recombinant attenuated bacterial cells as antigen carriers, which are capable of expressing a DNA molecule encoding a Helicobacter antigen. Apparently, recombinant attenuated bacterial cells expressing a Helicobacter antigen are capable of creating a qualitatively different immune response against the heterologous Helicobacter antigen than Helicobacter itself does against its own homologous antigen. Surprisingly, a non-protective immune response is thus transformed into an immune response protecting against Helicobacter infections. This unexpected observation renders it possible to use recombinant attenuated pathogens, e.g. bacterial cells, particularly Salmonella, as carriers for the screening of protective antigens, to apply the protective antigens identified in this manner in any vaccine against Helicobacter infections, and to use recombinant attenuated bacteria as carriers of protective antigens for the immunization against Helicobacter infections in humans and other mammals.

Thus, a subject matter of the present invention is a pharmaceutical composition comprising as an active agent an immunologically protective living vaccine which is a recombinant attenuated *Salmonella* cell which comprises at least one heterologous nucleic acid molecule encoding a *Helicobacter* antigen wherein said *Salmonella* cell is capable of expressing said nucleic acid molecule or capable of causing the expression of said nucleic acid molecule in a target cell, and wherein the *Helicobacter* antigen is urease, a urease subunit or an immunologically reactive fragment thereof.

Preferably the nucleic acid is a DNA molecule.

The *Salmonella* cell is a *Salmonella* aro mutant cell.

The nucleic acid molecule which is inserted into the pathogen codes for a Helicobacter antigen, preferably a H. felis, H. heilmanii or H. pylori antigen, more preferably a H. pylori antigen. The Helicobacter antigen can be a native Helicobacter polypeptide, an immunologically reactive fragment thereof, or an immunologically reactive variant of a native polypeptide or of a fragment thereof.

The nucleic acid molecules coding for Heliobacter antigens may be located on an extrachromosomal vector, e.g. a plasmid, and/or integrated in the cellular chromosome of the pathogen. When the pathogen is used as a vaccine, chromosomal integration usually is preferred.

Attenuated Salmonella cells can be used to transcribe and translate said nucleic acid molecule directly in the Salmonella cell or to deliver said nucleic acid molecule to the infected target cell, such that the DNA molecule is transcribed and/or translated by the eukaryotic target cell machinery. This indirect bacterial vaccination procedure, termed here as genetic vaccination, has been successfully used with Shigella as a carrier (Sizemore, D. R., Branstrom, A. A. & Sadoff, J. C. (1995) Attenuated Shigella as a DNA delivery vehicle for DNA-mediated immunization. Science 270:299-302).

The Helicobacter antigen is urease, a urease subunit or an immunologically reactive variant or fragment thereof.

It is also conceivable, however, that an intracellular antigen is used which can be presented on the surface, e.g. by autolytic release, and confers immunological protection.

The presentation of the Helicobacter antigens in the recombinant Salmonella cell according to the invention can be accomplished in different ways. The antigen or the antigens can be synthesized in a constitutive, inducible or phase variable manner in the recombinant pathogen. Concerning the constitutive or inducible synthesis of the Helicobacter antigens known expression systems can be referred to, as have been described by Sambrook et al., Molecular Cloning, A Laboratory Manual (1989), Cold Spring Harbor Laboratory Press.

Particularly preferred the antigens are presented in a phase variable expression system. Such a phase variable expression system for the production and presentation of foreign antigens in hybrid live vaccines is disclosed in EP-B-O 565 548, which is herein incorporated by reference. In such a phase variable expression system the nucleic acid molecule encoding the Helicobacter antigen is under control of an expression signal, which is substantially inactive in the pathogen, and which is capable of being activated by a spontaneous reorganization caused by a nucleic acid, e.g. DNA reorganization mechanism in the pathogen, e.g. a specific DNA inversion process, a specific DNA deletion process, a specific DNA replication process or a specific slipped-strand-mispairing mechanism.

A recombinant cell having a phase variable expression system is capable of forming two subpopulations A and B, wherein the division into said subpopulations occurs by spontaneous reorganization in the recombinant nucleic acid, wherein said subpopulation A is capable of infection and immunologically active per se, while subpopulation B, which is regenerated from subpopulation A, produces at least one heterologous Helicobacter antigen and acts immunologically with respect to said additional antigen.

The activation of the expression signal encoding the Helicobacter antigen can be directly accomplished by nucleic acid reorganization or, alternatively, indirectly accomplished by activation of a gene encoding a protein which controls the expression of the gene encoding the Helicobacter antigen. The indirect activation represents a system which allows the production of the Helicobacter antigen via a cascade system, which can be realized e.g. in that the gene directly controlled by DNA reorganization codes for an RNA polymerase which is specific for the promoter preceding the Helicobacter gene, or a gene regulator which in another specific manner induces the expression of the Helicobacter gene. In an especially preferred embodiment of the present invention the expression signal for the gene encoding the Helicobacter antigen is a bacteriophage promoter, e.g. a T3, T7 or SP6 promoter, and the activation of the expression signal is caused by a nucleic acid reorganization resulting in the production of a corresponding bacteriophage RNA polymerase in the pathogen.

The phase variable expression system can be adjusted to provide a preselected expression level of the Helicobacter antigen. This can be accomplished e.g. by modifying the nucleotide sequence of the expression signal, which is activated by the nucleic acid reorganization mechanism, and/or by inserting further genetic regulation elements.

The Helicobacter antigens can be produced in an intracellular, as well as in an extracellular manner in the pathogen according to the invention. For instance, autotransporter systems such as the IgA-protease system (cf. for instance EP-A-0 254 090) or the E. coli AIDA-1 adhesin system (Benz et al., Mol. Microbiol. 6 (1992), 1539) are suited as extracellular secretory system. Other suitable outer membrane transporter systems are the RTX-toxin transporters, e.g. the E. coli hemolysin transport system (Hess et al., Proc. Natl. Acad. Sci. USA 93 (1996), 11458-11463).

The pathogen according to the invention can contain a second heterologous nucleic acid, e.g. DNA molecule, which codes for an immunomodulatory polypeptide influencing the immune response quantitatively or qualitatively, apart from the nucleic acid molecule encoding the Helicobacter antigen. Examples of such immunomodulatory polypeptides are immune-stimulating peptides, cytokines like IL-2, IL-6 or IL-12, chemokines, toxins, such as cholera toxin B or adhesins.

The present invention also refers to a pharmaceutical composition comprising as an active agent a recombinant attenuated Salmonella cell as described above, optionally together with pharmaceutically acceptable diluents, carriers and adjuvants. Preferably, the composition is a living vaccine. The vaccination routes depend upon the choice of the vaccination vector. The administration may be achieved in a single dose or repeated at intervals. The appropriate dosage depends on various parameters such as the vaccinal vector itself, or the route of administration. Usually the dosage comprises about 10⁶ to 10¹² cells (CFU), preferably about 10⁸ to 10¹⁰ cells (CFU) per vaccination. Administration to a mucosal surface (e.g. ocular, intranasal, oral, gastric, intestinal, rectal, vaginal or urinary tract) or via the parenteral route (e.g. subcutaneous, intradermal, intramuscular, intravenous or intraperitoneal) might be chosen. A method for the preparation of the living vaccine comprises formulating the attenuated pathogen in a pharmaceutically effective amount with pharmaceutically acceptable diluents, carriers and/or adjuvants.

The pharmaceutical composition may be provided in any suitable form, e.g. a suspension in suitable liquid carrier, such as water or milk, a capsule, a tablet etc. In a preferred embodiment of the present invention the composition is a lyophilized product which is suspended in a liquid carrier prior to use.

Further, the present invention refers to a method for preparing a recombinant attenuated *Salmonella* cell as defined above, comprising the steps of a) inserting a nucleic acid molecule encoding a Helicobacter antigen into an attenuated *Salmonella* cell, wherein the recombinant attenuated *Salmonella* cell, e.g. a transformed *Salmonella* cell, is obtained, which is capable of expressing said nucleic acid molecule or is capable to cause expression of said nucleic acid molecule in a target cell and wherein the Helicobacter antigen is urease, a urease subunit or an immunologically reactive fragment thereof and b) cultivating said recombinant attenuated *Salmonella* cell under suitable conditions. The nucleic acid molecule encoding the Helicobacter antigen can be located on an extra- chromosomal plasmid. It is, however, also possible to insert the nucleic acid molecule into the chromosome of the *Salmonella cell.*

The invention will be further illustrated by the following figures and sequence listings.
- Fig. 1:: shows a schematic illustration of the urease expression vector pYZ97, whereon the genes coding for the urease subunits UreA and UreB are located under transcriptional control of the T7 promoter φ10. There is a ribosomal binding site (RBS) between the T7 promoter and the urease genes. Further, the plasmid exhibits an origin of replication (ori), a β-lactamase resistance gene (bla) and 4 T7 terminators in series.
Apart from the expression by the T7 promoter, a constitutive low level expression of the urease A and B subunits can also be brought about via a cryptic promoter, which is located upstream from the T7 promoter, on the plasmid pYZ97.
- Fig.2:: shows the nucleotide sequence of the transcriptional regulation region for urease expression and the beginning of the amino acid sequence of urease subunit A on plasmid pYZ97.
- Fig.3:: shows a schematic illustration of the T7 RNA polymerase (T7RNAP) expression cassettes pYZ88, pYZ84 and pYZ114, which can be integrated into the chromosomes of bacteria.
In the high-expression cassette pYZ88 the lambda PL promoter is located in inverse orientation, upstream from the T7RNAP gene. A gene for the temperature-sensitive repressor cI 857 (cI) is under control of this promoter. A terminator of the bacteriophage fd (fdT) is situated upstream from the cI gene. The gin gene (Mertens, EMBO J. 3 (1984), 2415-2421) codes for a control enzyme of a DNA reorganization mechanism. A DNA sequence coding for the tRNA Arg is located downstream from the gin gene.
In phase A the PL promoter responsible for the expression of the T7RNAP gene is directed in the direction of the cI857 gene and the gin gene. The consequence of this is that an active repressor is formed at the permissive temperature of 28°C and reduces the transcription from the PL promoter. At a higher temperature the transcription of the PL promoter is increased, since the repressor is inactivated at least partially under such external influences. The temperature-dependent increase in the transcription also causes a corresponding increase in the expression of the following gin gene, which as a control enzyme catalyses the inversion of the PL promoter and the transition in phase B, in which the T7RNAP gene is expressed.
In the high-expression system pYZ88 a further fdT transcription terminator is located between a kanamycin-resistance gene (km) and the promoter of this gene. In this manner, the synthesis of an anti-sense RNA, inversely orientated to the T7RNAP gene, which normally contributes to the reduction of the T7RNAP expression, is reduced. This results in a high expression of the T7RNAP.
In the medium-expression system pYZ84 a transcription terminator (fdT) is located between the PL promoter and the start of the T7RNAP gene. In this manner the expression of the T7RNAP mRNA is reduced. Additionally, the anti-sense RNA affects the T7RNAP translation. Therefore, only a medium expression occurs.
In the low-expression system pYZ114 a deletion of 100 bp in PL is additionally introduced (Δ PL). In this manner the activity of the PL promoter is reduced to a high extent, which leads to a lower T7RNAP expression and thus to a reduction of the UreA/B gene expression. In this construct the effect of the cryptic promoter on pYZ97 is already observed.

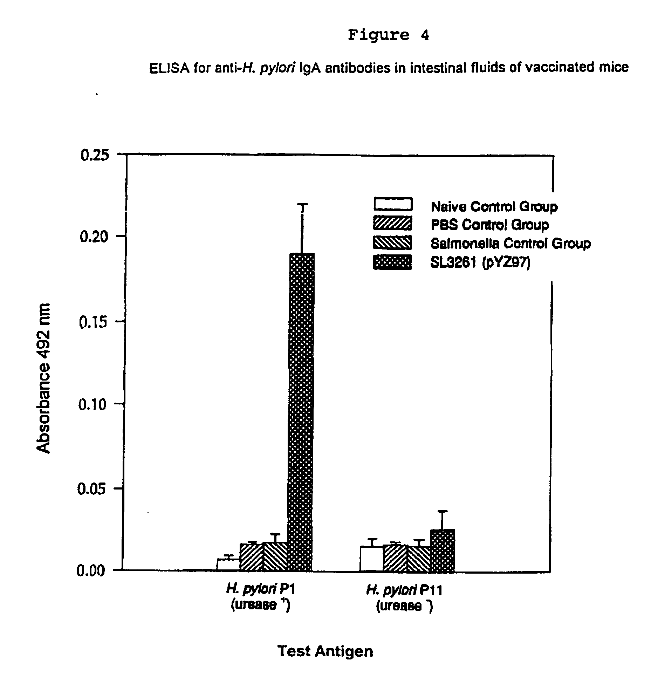

- Fig.4:: shows the results of an ELISA for anti-H.pylori antibodies in intestinal fluids of vaccinated mice.

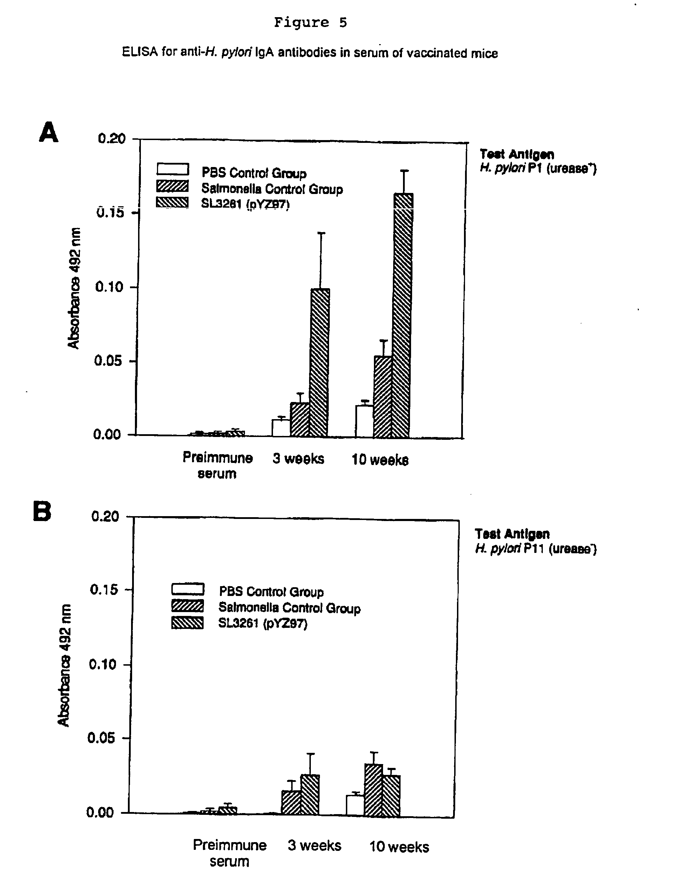

- Fig.5:: shows the results of an ELISA for anti-H.pylori antibodies in the serum of vaccinated mice.

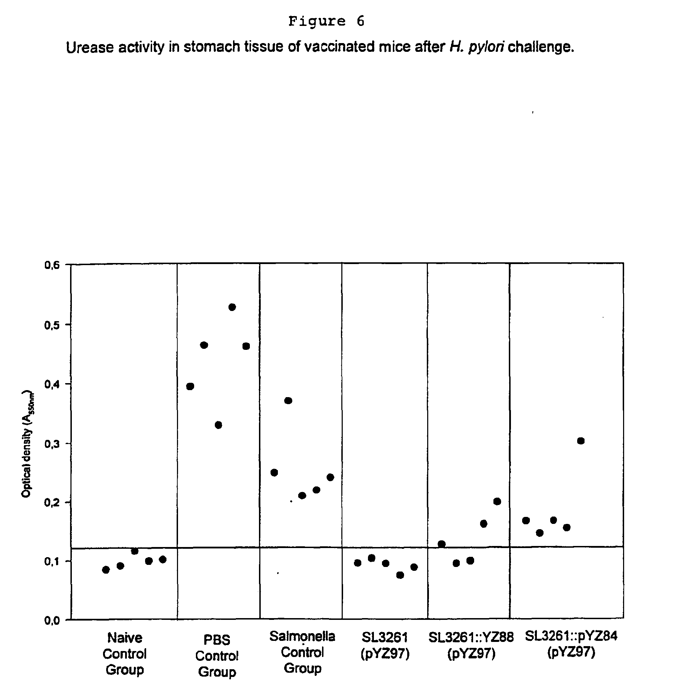

- Fig.6:: shows the urease activity in the stomach tissue of vaccinated mice after H.pyroli challenge.

SEQ ID NO. 5 and 6 show the nucleotide sequence of the transcriptional regulation region for urease expression and the beginning of the amino acid sequence of urease subunit A on plasmid pYZ97.

### Experimental part

### Example 1

### Cloning of the ureA and ureB genes.

The structural genes encoding the urease, *ureA* and *ureB*, have been genetically cloned from chromosomal DNA of a clinical specimen P1 (formerly 69A) isolated at the University of Amsterdam and provided by Dr. Jos van Putten. The genes were isolated by a PCR-approach using the primer pair YZ019 (5'-GGAATTCCATATGAAACTGACTCCCAAAGAG-3') and RH132 (5'-CTGCAGTCGACTAGAAAATGCTAAAGAG-3') for amplification. The sequence of the primers was deduced from GenBank (accession numbers M60398, X57132). The DNA sequence of primer YZ019 covered the nucleotides 2659-2679 of the published sequence and further contained a translational regulatory sequence (down stream box; Sprengart, M. L. et al., 1990, Nuc. Acid. Res. 18:1719-1723) and a cleavage site for *NdeI.* The DNA sequence of primer RH132 covered the nucleotides 5071-5088 of the published sequence and a cleavage site for *SalI.* The amplification product was 2.4 kbp in size comprising the complete coding region of *ureA* and *ureB* genes without the original transcriptional start and termination sequences from the *Helicobacter* chromosome. The purified PCR-fragment was digested with *NdeI* and *SalI* and inserted into the corresponding cloning sites of T7 expression plasmid pYZ57 to yield the plasmid pYZ97.

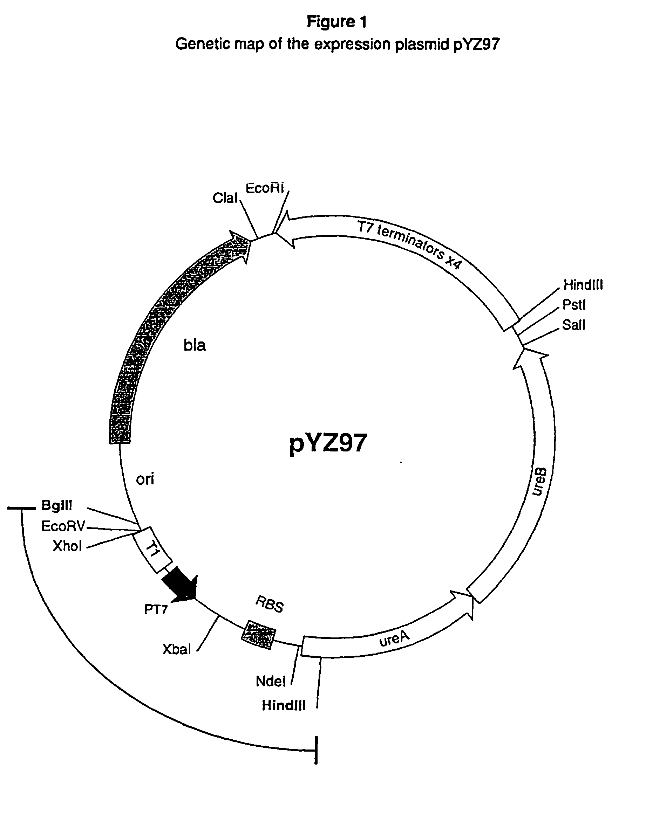

pYZ57 was originally derived from plasmid pT7-7, which was described by Tabor (1990, In Current Protocols in Molecular Biology, 16.2.1-16.2.11. Greene Publishing and Wiley-Interscience, New York). Two terminator fragments were introduced into the pT7-7 backbone at different sites by the following strategy: (1) The tandem T7 terminators. A 2.2 kbp *EcoRI*/*HindIII* fragment was excised from pEP12 (Brunschwig & Darzins, 1992, Gene, 111:35-41) and the purified fragment ligated with predigested pBA (Mauer, J. et al., 1997, J. Bacteriol. 179:794-804). The ligation product was digested with *HindIII* and *ClaI.* The resulting 2.2 kbp *HindIII*/*ClaI* fragment was subsequently inserted into predigested pT7-7. (2) The T1 terminator. A 230 bp *HpaI*/*NdeI*-fragment was excised from plasmid pDS3EcoRV (provided by Dr. H. Bujard; ZMBH, Heidelberg). The fragment was then further treated with *Klenow* to generate blunt ends. The purified rrnBT1 fragment was inserted into the previous pT7-7 derivative, predigested with *BglII* and subsequently bluntended by Klenow treatment. Figure 1 describes the completed vector pYZ97 used for the expression of the urease genes coding for urease subunits UreA and UreB in *S*. *typhimurium.* As indicated in Figure 1, the urease genes can be controlled by the T7 promoter φ10. The ribosome binding site (RBS) is located between the T7 promoter and the urease genes. Further, the plasmid exhibits an origin of replication (*ori*) and a β-lactamase resistance gene (bla).

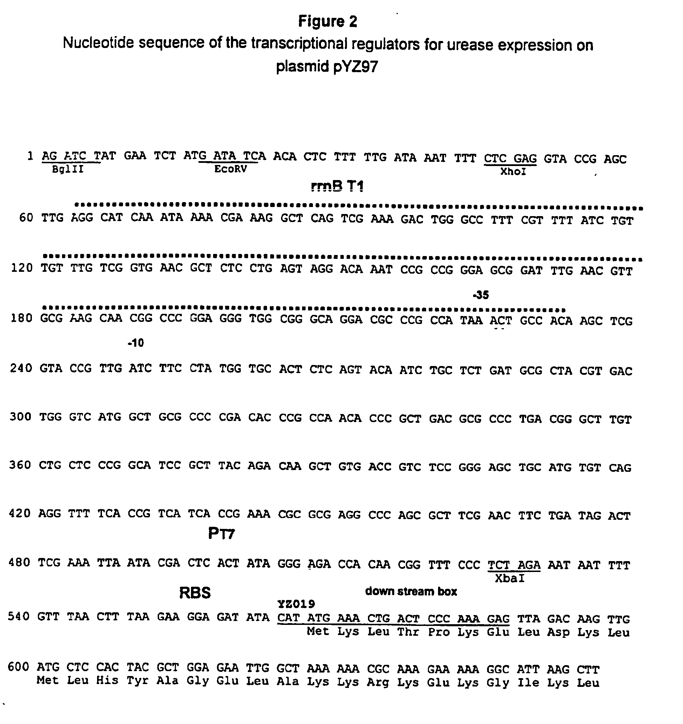

Apart from the expression controled by the T7 promoter, a constitutive moderate level expression of the urease A and B subunits does occur from a promoter driven by *Salmonella* RNA polymerase. The promoter is located upstream from the T7 promoter, on the plasmid pYZ97. For detailed molecular analysis, the purified *BglII*/*HindIII*-fragment of pYZ97 was subcloned into the pCR-Script^{™} SK(+)kit (Stratagene) and subjected to DNA-sequencing. The sequence data confirmed the various elements in their completeness (see Figure 2 and SEQ ID NO.5 and 6) : part of the ureA gene, the down-stream box, the RBS, the T7 promoter and the T1 terminator (rrnBT1). The sequence analysis also disclosed the region between the T1 terminator region and the T7 promoter where the *Salmonella* RNA polymerase promoter is localised. The sequence data suggests a location of this constitutive promoter between nucleotides 222 - 245 which have been deduced from structural predictions (Lisser & Margalit, 1993, Nuc. Acid. Res. 21:1507-1516).

### Example 2

### Immunological protection by administration of live vaccine

### Materials and Methods

Bacterial strains: S. typhimurium SL3261 live vector vaccine strain was used as a recipient for the recombinant H. pylori urease plasmid constructs. S. typhimurium SL3261 is an aroA transposon mutant derived from S. typhimurium SL1344 wild type strain. S. typhimurium SL3261 is a non-virulent strain that gives protection to mice against infection with wild type S. typhimurium after oral administration (Hoiseth and Stocker (1981) Supra). S. typhimurium SL3261 and derivatives thereof, which contain the urease expression plasmid pYZ97 (extrachromosomal) and the T7RNAP expression cassettes pYZ88, pYZ84 or pYZ114, respectively (integrated into the chromosome) are indicated in table 1. Luria broth or agar was used for bacterial growth at 28°C. H. pylori wild type strain grown at 37°C on serum plates was used for the challenge experiments.

Immunization of mice: Four weeks Balb/c mice purchased from Interfauna (Tuttlingen, Germany) were adapted two weeks in an animal facility before being used for experimentation. 150 µl of blood was taken retroorbitally from all mice to obtain preimmune serum. Retroorbital bleedings were repeated from all immunized mice 1 week and 3 weeks after immunization.

Eight groups of 5 mice including controls were used in this study (table 2). Group A, the naive control group, was not immunized with Salmonella neither challenged with wild type H. pylori. The rest of the groups were all orally immunized. Group B, a negative control group, did not receive Salmonella and was challenged with H. pylori. Mice from groups C to G were immunized with Salmonella vaccine strains and challenged with H. pylori. The last group H received recombinant urease B in combination with cholera toxin and was also challenged.

Prior to immunizations mice were left overnight without solid food and 4 hours without water. 100 µl of 3% sodium bicarbonate were given orally using a stainless steel catheter tube to neutralize the stomach pH. Then mice from group B received 100 µl PBS and mice from groups C to G received 1.0 x 10¹⁰ CFU of Salmonella in a 100 µl volume. Mice from group H received four times 100 µl of a mixture of recombinant H. pylori UreaseB plus cholera toxin, one dose every week. After every immunization water and food were returned to the mice.

H. pylori challenge: Four weeks after the first oral immunization mice from groups B to H were challenged with H.pylori. Mice were left overnight without solid food and without water 4 hours prior to the challenge. 100 µl of 3% sodium bicarbonate were given orally to the mice using a stainless steel catheter tube, followed by an oral dose of 5.0 x 10⁹ CFU/ml of Helicobacter pylori. Water and food were returned to the mice after the challenge.

Collection of blood and tissues from mice: Twelve weeks after the first immunization the mice were left overnight without food and subsequently sacrificed for analysis of protection and immune response. The mice were anaesthetized with Metoxyfluorane for terminal cardiac bleeding and prior to sacrifice by cervical dislocation. Under aseptic conditions, spleen and stomach were carefully removed from each mouse and placed on ice in separate sterile containers until further processing. Large and small intestine were obtained for further isolation of the intestinal fluid.

Processing of stomach and measurement of urease activity: The degree of H. pylori colonisation in the mouse stomach was measured by the presence of active urease in the tissue. The Jatrox-test (Röhm-Pharma GmbH, Weiterstadt, Germany) was used according to the suppliers' directions. Stomach mucosa was exposed and washed with PBS, half of the antral portion of the stomach was immediately placed inside an Eppendorf tube containing the substrate for measurement of urease activity. Absorbance at 550 nm was measured after tubes were incubated for 4 hours at room temperature. The rest of the stomach tissue was stored at -20°C for further treatments. The urease activity values obtained from the stomach of naive mice, which did not undergo immunization or challenge, were used to create a base line to indicate the absence of H. pylori infection and therefore protection.

**Table 1**

| **UreA and UreB expressing S. typhimurium vaccine strains** | | |
|---|---|---|
| Strains | Urease Expression | Source |
| S. typhimurium SL3261 | Negative | Hoiseth and Stocker |
| S. typhimurium SL3262 pYZ97 | Constitutive Low | this study |
| S. typhimurium SL3261::pYZ88pYZ97 | High T7-induced expression | this study |
| S. typhimurium SL3261::pYZ84pYZ97 | Medium T7-induced expression | this study |
| S. typhimurium SL3261::pYZ114pYZ97 | Low T7-induced expression | this study |

**Table 2**

| **Mice groups used for immunization** | | |
|---|---|---|
| Group | Immunogen | No. of oral immunizations |
| A | None | 0 |
| B | PBS oral immunization | 1 |
| C | S. typhimurium S3261 | 1 |
| D | S. typhimurium S3261 pYZ97 | 1 |
| E | S. typhimurium S3261::pYZ88pYZ97 | 1 |
| F | S. typhimurium S3261::pYZ84pYZ97 | 1 |
| G | S. typhimurium S3261::pYZ114pYZ97 | 1 |
| H | Urease B plus cholera toxin | 4 |

### Results:

In the control mice (groups B and C) 100% infection with H. pylori was observed. In the mice immunized with recombinant attenuated pathogens (groups D, E, F, G) between 0% and 60% infection (100% to 40% protection) was observed. Immuno-protection did not correlate with humoral anti-UreA and UreB response, suggesting that, in addition to humoral immunity, cellular immunity is critical for protection against H. pylori infection. The results indicate that oral immunization of mice with UreA and UreB delivered by S. typhimurium attenuated strain is effective to induce high levels of protection against H. pylori colonisation.

In the mice immunized with recombinant urease B plus cholera toxin considerably higher levels of urease activity were observed under said experimental conditions than when administering the recombinant attenuated pathogens according to the invention.

The results of the urease test have been illustrated in table 3.

**Table 3**

| Group | Mouse | E_{550nm, 4h} | E₄ₕ - E_{control} | E_{cont} ^{°3} | Dilution |
|---|---|---|---|---|---|
| A | 1 | 0,085 | -0,022 | -0,066 | 200µl+400µl |
| A | 2 | 0.091 | -0,016 | -0,048 | 200µl+400µl |
| A | 3 | 0,116 | 0,009 | 0,027 | 200µl+400µl |
| A | 4 | 0,099 | -0,008 | -0,024 | 200µl+400µl |
| A | 5 | 0,101 | -0,006 | -0,018 | 200µl+400µl |
| Control | | 0.107 | 0 | 0 | 200µl+400µl |
| B | 1 | 0,394 | 0,292 | 0,876 | 200µl+400µl |
| B | 2 | 0,464 | 0,362 | 1,086 | 200µl+400µl |
| B | 3 | 0.329 | 0.227 | 0,681 | 200µl+400µl |
| B | 4 | 0.527 | 0.425 | 1,275 | 200µl+400µl |
| B | 5 | 0,462 | 0,36 | 1.08 | 200µl+400µl |
| Control | | 0,102 | 0 | 0 | 200µl+400µl |
| C | 1 | 0,248 | 0,145 | 0,435 | 200µl+400µl |
| C | 2 | 0,369 | 0,266 | 0,798 | 200µl+400µl |
| C | 3 | 0,209 | 0,106 | 0,318 | 200µl+400µl |
| C | 4 | 0,219 | 0,116 | 0,348 | 200µl+400µl |
| C | 5 | 0.24 | 0,137 | 0,411 | 200µl+400µl |
| Control | | 0,103 | 0 | 0 | 200µl+400µl |
| D | 1 | 0.143 | 0,002 | 0,004 | 300µl+300µl |
| D | 2 | 0,156 | 0,015 | 0,03 | 300µl+300µl |
| D | 3 | 0,142 | 0,001 | 0,002 | 300µl+300µl |
| D | 4 | 0,114 | -0,027 | -0,054 | 300µl+300µl |
| D | 5 | 0,133 | -0,008 | -0,016 | 300µl+300µl |
| Control | | 0,141 | 0 | 0 | 300µl+300µl |
| E | 1 | 0.127 | 0,027 | 0,081 | 200µl+400µl |
| E | 2 | 0,094 | -0,006 | -0,018 | 200µl+400µl |
| E | 3 | 0,099 | -0,001 | -0,003 | 200µl+400µl |
| E | 4 | 0,161 | 0,061 | 0,183 | 200µl+400µl |
| E | 5 | 0,198 | 0.098 | 0,294 | 200µl+400µl |
| Control | | 0,1 | 0 | 0 | 200µl+400µl |
| F | 1 | 0,166 | 0,025 | 0,05 | 300µl+300µl |
| F | 2 | 0,145 | 0,004 | 0,008 | 300µl+300µl |
| F | 3 | 0,166 | 0,025 | 0,05 | 300µl+300µl |
| F | 4 | 0,154 | 0,013 | 0,026 | 300µl+300µl |
| F | 5 | 0,301 | 0,16 | 0,32 | 300µl+300µl |
| Control | | 0,141 | 0 | 0 | 300µl+300µl |
| G | 1 | 0,084 | -0,019 | -0,057 | 200µl+400µl |
| G | 2 | 0,087 | -0,016 | -0,048 | 200µl+400µl |
| G | 3 | 0,269 | 0,166 | 0,498 | 200µl+400µl |
| G | 4 | 0,085 | -0,018 | -0,054 | 200µl+400µl |
| G | 5 | 0.092 | -0,011 | -0,033 | 200µl+400µl |
| Control | | 0,103 | 0 | 0 | 200µl+400µl |
| H | 1 | 0.638 | 0,531 | 1,593 | 200µl+400µl |
| H | 2 | 0.282 | 0,175 | 0,525 | 200µl+400µl |
| H | 3 | 0,141 | 0,034 | 0,102 | 200µl+400µl |
| H | 4 | 0,135 | 0,028 | 0,084 | 200µl+400µl |
| H | 5 | 0,171 | 0,064 | 0,192 | 200µl+400µl |
| Control | | 0,107 | 0 | 0 | 200µl+400µl |

### Example 3

### Construction and molecular analysis of various recombinant S. typhimurium strains expressing ureA/ureB subunits.

### Description of the S. typhimurium strains used for immunization experiments.

*S. typhimurium* SL3261(pYZ97) (**construct A**): *S*. *typhimurium* SL3261 live vaccine vector strain was used as a recipient for the recombinant urease plasmid construct pYZ97.

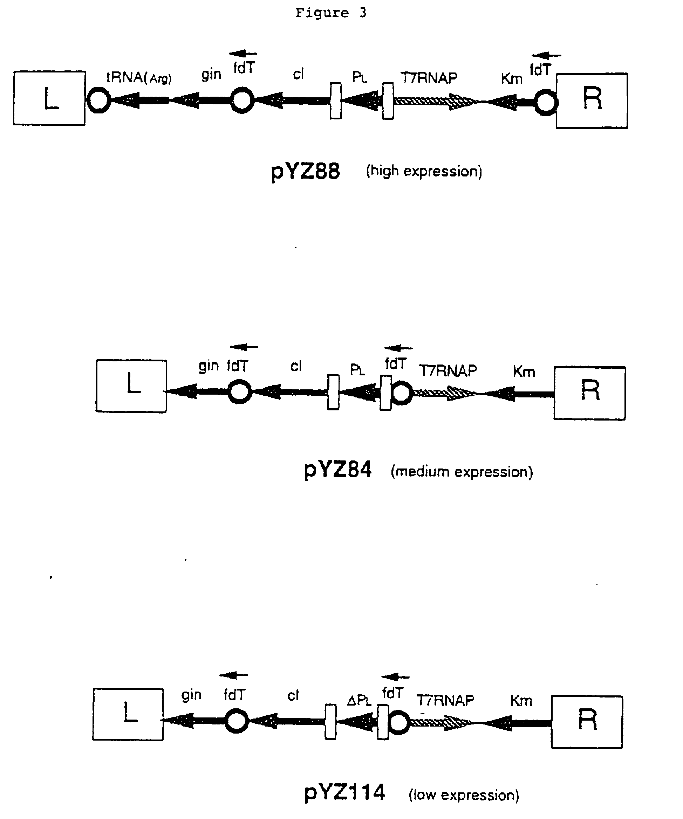

*S. typhimurium* SL3261::YZ Series (pYZ97) (construct B): These carrier strains are a derivative of *S. typhimurium* SL3261 which has been equipped with the T7 RNA polymerase (T7RNAP) expression cassettes schematically presented in Figure 3. These expression cassettes encode the gene for T7RNAP which is expressed in a 2-phase modus (ON/OFF) as disclosed in a previous invention of Yan et al. ("Two phase system for the production and presentation of foreign antigens in hybrid live vaccines", PCT/EP91/02478). The cassette can be integrated into the chromosome of bacteria and provide the cell in ON-position with optimal amount of T7RNAP for activation of T7RNAP-dependent expression plasmids such as pYZ97.

The principle of the YZ84 cassette is an invertible lambda PL promoter placed on a fragment that is inverted by the phage Mu invertase Gin (Yan & Meyer, 1996, J. Biotechnol. 44:197-201). Dependent on the orientation of the PL promoter either the gin gene (OFF-position) or the T7RNAP gene (ON-position) is expressed. The following regulatory elements have been included in YZ84: (1) The temperature-sensitive cIₜₛ lambda repressor (cI) which represses the PL promoter at 28°C and dissociates at 37°C. (2) The phage fd terminator (fdT) reduces expression of gin gene in order to achieve moderate inversion rates of the PL promoter on the invertible fragment.

The 2-phase expression system enables high expression rates of foreign antigens, such as the urease subunits A and B. It is well known that high expression rates of foreign antigens reduce viability of *Salmonella* carrier thus diminishing immune response and consequently the protective potential. It was shown that the 2-phase system has a natural competence to improve survival of recombinant *Salmonella* which express large amounts of foreign antigen. In construct B, expression of the *ureA* and *ureB* genes is mainly under the control of the strong T7 promoter resulting in high production of the urease subunits. If the T7RNAP expression cassette is in OFF-position and no T7RNAP is present, the *ureA* and *ureB* genes are constitutively expressed in moderate range by the *Salmonella* promoter.

### Analysis of ureA/B subunits produced by the various S. typhimurium strains used for immunization experiments.

*Salmonella* constructs A and B were first analyzed by SDS-polyacrylamide gels for expression of UreA and UreB. The recombinant strains were grown at 37°C in liquid Luria Broth supplemented with 100µg/ml Ampicillin starting from an over night culture. The bacteria were harvested at logarithmic growth phase by centrifugation and the cell pellet was resuspended in 10mM Tris-HCl and 10 mM EDTA, (pH 8.0) and cell-density adjusted to standard A₅₉₀=1.0 in all probes. The bacterial suspension was mixed with the same volume of SDS-sample buffer (Sambrook, J. et al. 1989. Molecular cloning: a laboratory manual. 2nd ed. Cold Spring Harbor Laboratory Press, Cold Spring Harbor, N.Y.) and boiled for 5 min. 20 µl of suspension were loaded onto two SDS-10 % polyacrylamide gels; one of the gels was stained with Coomassie blue stain and the other was electroblotted onto a nitrocellulose membrane and further processed for immunoblotting. The nitrocellulose membrane carrying the transferred proteins was blocked for 45 min at room temperature in 10 (v/w)% non-fat milk Tris-buffer-saline (TBS) (TrisHCl 100mM, NaCl 150mM, pH 7.2). After three washes in TBS-0,05 (v/v)% Tween-20, a 1:2000 dilution of rabbit anti-UreB antibody (AK 201) in 5 (w/v)% non-fat milk-TBS was added to the strip and incubated overnight at 4°C. Serum was obtained from rabbit immunised with recombinant urease B subunit purified via affinity chromatography. The membrane was washed three times for 10 min with 0,05 (v/v)% Tween-20 in PBS, and further incubated in 5 (w/v)% non-fat milk-TBS with goat anti-rabbit IgG antibody horse radish peroxidase conjugate for 45 min at room temperature. After three washes with 0.2 (v/v)% Tween-20 as above, the membrane was developed using the ECL kit (Amersham, Germany) following the recommendations of the suppliers.

**Construct A**: Proteins of 67 kDa and 30 kDa were observed in the Coomassie stained gel of the whole cell lysate of construct A (*S. typhimurium* strain SL3261(pYZ97); these sizes correlate very well with those of UreB and UreA, respectively. Such proteins were absent in the control lanes containing the *S. typhimurium* SL3261 strain. Immunoblot analysis of the same protein samples using a rabbit anti-UreB antibody confirmed the 67 kDa protein observed in the Coomassie stained gel as UreB. Expression of *ureB* from *S. typhimurium* strain SL3261(pYZ97) was also examined at different phases of growth by incubating at 37°C for 2, 6 and 11 hours, respectively. Expression of *ureB* was observed in all phases of growth including in the stationary phase; although, higher expression was observed at early phases of growth. The results obtained with strain SL3261(pYZ97) indicate that UreA and UreB proteins are non-toxic for *Salmonella* and can be expressed at 37°C at any phase of bacterial growth.

**Construct B**: Similar analysis were performed with construct B. The comparison of both constructs in SDS-PAGE analysis reveals that construct B is the more efficient producer whilst construct A has moderate expression of *ureA* and *ureB.* In the course of bacterial growth of construct B, the expression of *ureA* and *ureB* is constantly high over a longer time period even without antibiotic selection. This confirms the exceptional productivity of construct B in comparison to construct A.

In summary, our data indicate that UreA and UreB from *H*. *pylori* can be expressed in *S. typhimurium* without causing adverse effects to the bacteria, and are, therefore, suitable for animal protection experiments when delivered by *Salmonella* carriers.

### Plasmid-stability

Plasmid stability is essential to assure stable expression of antigens coded by genes which have been cloned into such plasmids.

**In vitro plasmid stability.** The ampicillin resistance marker present on plasmid pYZ97 and absent in the plasmidless *S. typhimurium* strain SL3261 was used as an indicator of plasmid stability. *S. typhimurium* strain SL3261 was grown in LB liquid medium at 28°C for up to 100 generations as described previously (Summers, D. K. and D. J. Sherrat. 1984. Cell. 36:1097-1103). Every ten generations, the number of ampicillin resistant CFU was determined from the total number of colony forming units (CFU) of *Salmonella* by plating equal number of bacterial dilutions on plain LB-agar plates and LB-agar plates supplemented with 100 µg/ml ampicillin.

**Plasmid stability in vivo**. Plasmid stability in vivo was analyzed by examining total CFU and ampicillin resistant CFU from mice spleen, two and seven days after oral infection of mice with 5.0 X10⁹ CFU of. *S. typhimurium* SL3261(pYZ97). Mice were orally infected with *Salmonella* as described above. Two days and seven days after infection mice were sacrificed under metoxyfluorane anesthesia, and the spleen was removed aseptically for further processing. The spleen was dissected in small pieces in a petri dish, mixed with 1 ml ice-cold ddH₂O, and passed several times through a 18 gauge needle to suspend the spleen cells. The cell suspension was then plated on LB-agar plates with or without 100 µg/ml ampicillin. Plates were incubated at 37°C overnight and colonies counted the next day.

Plasmid stability *in vivo* was analyzed after infecting mice with one oral dose of 5.0 x 10⁹ CFU of *S*. *typhimurium* SL3261(pYZ97). Mice spleens were taken two and seven days after infection, and plated on LB-agar plates for examination of total CFU and ampicillin resistant CFU. 2.0 x 10⁴ ampicillin resistant CFU were isolated from the spleens after 48 h (Table 4). The CFU counts decreased to 56 at 7 days after immunization, but again, all were ampicillin resistant. The data indicate that plasmid pYZ97 is stable in *Salmonella* under *in vitro* and *in vivo* conditions and is suitable for the evaluation of urease subunits as protective antigens against mouse stomach colonization by *H. pylori.* The low recovery of *Salmonella* strain SL3261 seven days after infection confirms the attenuation of this strain which allows its safe use for delivery of urease into mice.

**Table 4**

| **Recovery of *S. typhimurium* SL3261pYZ97 strain from mouse spleens and evaluation of pYZ97 plasmid stability *in vivo.*** | | |
|---|---|---|
| Time after infection | Total CFU^{a} | Percentage of Amp^{r} CFU^{b} |
| 2 days | 2.0X10⁴ | 100 |
| 7 days | 56 | 100 |

| | | |
|---|---|---|
| ^{a} Number of CFU of *S*. *typhimuriun* isolated on LB plates without antibiotics from the mouse spleens two and seven days after mice had been orally inoculated with 5.0X10⁹ CFU of *S*. *typhimuriun* strain SL3261 (pYZ97). ^{b} Percentage of ampicillin resistant CFU from the total No. of CFU isolated from mouse spleens. | | |

**Table 5**

| **Examination of urease activity and streptomycin resistant *H.pylori* in stomach antrum from mice immunized with UreA and UreB-expressing *Salmonella.*** | | | |
|---|---|---|---|
| **Mice group** | **No.** | **Urease activity^{a}** | **CFU^{b}** |
| Naive Control Group | 5 | 0.058 ± 0.004 | 0 ± 0 |
| PBS Control Group | 5 | 0.427 ± 0.059 | 2.7X10³ ± 1.0X10³ |
| SL3261pYZ97^{c} | 5 | 0.057 ± 0.006 | 62.6 ± 97.3 |

| | | | |
|---|---|---|---|
| ^{a} Urease activity is a mean value ± standard deviation. ^{b} Determination of CFU of the streptomicin resistant *H. pylori* P76 strain was carried out by plating a section of antrum stomach on serum plates supplemented with 200 µg/ml of streptomycin. *H. pylori* were recognized based on colony morphology, urease activity, and light microscopy examination. Values correspond to CFU ± standard deviation. ^{c} Mice immunized with *S. typhimurium* SL3261 (pYZ97) expressing *ureA* and *ureB from H. pylori* as described in Materials and Methods. | | | |

### Example 4

### Protection experiments with the various recombinant S. typhimurium strains expressing ureA/ureB subunits in H. pylori mouse model.

### Description of the Helicobacter pylori strains used for the experiments

Urease-deficient *H. pylori* P11 strain is a derivative of P1, generated by transposon shuttle mutagenesis using the Tn*Max*5 mini-transposon as disclosed in the invention of Haas et al. ("Verfahren zur Identifizierung sekretorischer Gene aus *Helicobacter pylori";* PCT/EP96/02544). Insertion of Tn*Max*5 has been mapped at the 3'-end of the ureA gene resulting in a defect expression of ureA and ureB due to transcriptional coupling of both genes.

Mouse-adapted *H. pylori* P49 strain was originally established by Dr. J. G. Fox (MIT, Boston, MA) from a feline isolate. *H. pylori* P76 strain is a streptomycin-resistant derivative of P49 generated by homologous recombination with chromosomal DNA from streptomycin-resistant *H*. *pylori* strain NCTC11637 as described by P. Nedenskov-Sorensen (1990, J. Infect. Dis. 161: 365-366).

All *H*. *pylori* strains were grown at 37°C in a microaerobic atmosphere (5% O₂, 85% N₂, and 10% CO₂) on serum plates (Odenbreit, S. et al. 1996. J. Bacteriol. 178:6960-6967) supplemented with 200 µg/ml of streptomycin when appropriate.

### Prophylactic immunization experiments with mice.

Immunization experiments were carried out to test the ability of UreA and B delivered by *Salmonella* to protect mice from stomach colonization by *H*. *pylori.* In total, 5 independent immunisation experiments have been performed. Each experiment consisted of 5 groups each with 5 mice: (1) **naive** control group was mice neither immunized with *Salmonella* nor challenged with wild type *H. pylori* P49 or the streptomycin resistant derivative strain P76; (2) **PBS control group** was non-immunized mice that received PBS and were challenged orally with *H. pylori*; (3) **Salmonella control group** was mice immunized with attenuated *S. typhimurium* SL3261 strain alone and challenged with *H*. *pylori;* and (5) the **vaccine group** was the mice immunized with appropriate recombinant *S. typhimurium* construct (A + B) expressing UreA and UreB and challenged with *H. pylori.*

Prior to immunizations, mice were left overnight without solid food and 4 hours without water. 100 µl of 3% sodium bicarbonate were given orally using a stainless steel catheter tube to neutralize the stomach pH. Immediately after stomach neutralization, mice from the **PBS control group** received 100 µl PBS, and mice from the ***Salmonella* control group** and ***Salmonella* vaccine group**, received 5.0 X 10⁹ CFU of *S*. *typhimurium* strain SL3261 and the various recombinant constructs, respectively, in a total volume of 100 µl. Water and food were returned to the mice after immunization.

Four weeks after the oral immunization, mice from the **PBS control-, *Salmonella* control-** and **vaccine-groups** were challenged with 1.0X10⁹ CFU of *H. pylori*. Mice were left overnight without solid food and without water 4 hours prior to the challenge. 100 µl of 3% sodium bicarbonate were given orally to mice using a stainless steel catheter tube, followed by an oral dose of 1.0 X 10⁹ CFU/ml of *H. pylori* strains P49 or P76 . Water and food were returned to mice after challenge.

### Example 5

Immunological analyses of protection experiments with the various recombinant *S*. *typhimurium* strains expressing ureA/ureB subunits in *H. pylori* mouse model

### Collection of blood and intestinal fluid from mice for serological analyses.

Antibody responses were evaluated from all mice using serum and intestinal fluid. 150 µl blood were collected retroorbitally before immunization and three weeks after immunization, before *Helicobacter* infection. The final bleeding was carried out 11 weeks after *Salmonella* immunization (6 weeks after challenge infection) by terminal cardiac puncture under metoxyfluorane anesthesia. The small intestines were taken from mice at the end of experiment and processed as described before (Elson, C. O. et al 1984. J. Immunol. Meth. 67:101-108) with minor modifications. Briefly, the content of intestines was removed by passing 2 ml of 50mM EDTA pH 7.5 (Riedel) containing 0,1mg/ml Soybean trypsin inhibitor (Sigma). The volume was adjusted to 5 ml with 0.15 M NaCl. The samples were vortexed vigorously, centrifuged 10 min at 2,500 rpm (Heraeus, Germany), and supernatant supplemented with 50 µl of 100 mM phenylmethylsulfonylfluoride (PMSF) (Serva) in 95% ethanol, followed by centrifugation at 13,000rpm for 20 min at 4°C (Hermes). Supernatants were supplemented with 50 µl of 100 mM PMSF and 50 µl of 2% sodium azide (Merck) and left on ice 15 min before addition of 250 µl of 7% bovine serum albumine (Biomol). The samples were frozen at -20°C until further use.

### Analysis of anti-urease antibodies in mouse sera and intestinal mucosa by ELISA.

Oral immunization with *Salmonella* is known to elicit IgA antibody responses. The IgA response against urease subunits in mice immunized with *S*. *typhimurium* construct A + B and in control mice was assessed by ELISA. A soluble extract of *H*. *pylori* P1 and its urease-deficient mutant derivative strain P11 was prepared in phosphate-buffer-saline by sonicating five times with a sonifier (Branson , Danbury, Conn.) at 5 sec intervals (35 % pulses) for 45 sec. This suspension was centrifuged at 13,000 rpm (Heraeus, Germany) for 10 min at 4°C to remove intact cells. The supernatant was used as antigen after determination of the protein content using the BioRad kit. 96-well microtiter plates (Nunc, Germany) were coated with 50 µl aliquot of 50 µg/ml of antigen in sodium carbonate-bicarbonate buffer pH 9.6 and incubated overnight at 4°C. The wells were blocked with 1.0 (w/v)% non-fat milk in Tris-buffer-saline (TBS) for 45 min at room temperature and washed three times with TBS-0.05% Tween-20. The assays, which were performed in triplicate, used 50 µl of serum or gut washing diluted 1:100 or 1:2 respectively in 0.5 (w/v)% non-fat milk-TBS added to the wells and left overnight at 4°C. The wells were then washed three times with TBS-0.05% Tween 20, and a 1:3000 dilution of a goat anti-mouse IgA horse-radish peroxidase-conjugate (Sigma) was added to all wells and incubated overnight at 4°C. The color reaction was developed by incubation at 37°C for 30 min with an orthophenylendiamine substrate in sodium acetate buffer and hydrogen peroxide. The reaction was stopped with 10 N H₂SO₄ and the A₄₉₂ was determined in an ELISA reader (Digiscan, Asys Hitech GmbH, Austria).

**Mucosal antibodies: (Construct A)** Intestinal fluid was taken from each sacrificed mouse at the end of the experiment (six weeks after the *H. pylori* challenge) and tested for the presence of anti-urease antibodies by using total cell extracts of *H. pylori* wild type (P1) and urease deficient mutant strains (P11). As shown in Fig. 4, the IgA antibody response against the wild type *H. pylori* extract was around 10-fold higher in immunized mice versus non-immunized or naive mice. The mucosal IgA antibody response against the urease-deficient *H. pylori* mutant was very low in all groups of mice indicating that most of the intestinal IgA antibody response in immunized mice was directed against urease.

**Serum antibodies: (Construct A)** The levels of serum IgA antibodies against a wild type and an urease-deficient *H*. *pylori* were examined prior to immunization, 3 weeks after immunization (before challenge) and 10 weeks after immunization (6 weeks after challenge with *H. pylori).* As shown in Fig. 5 panel A, the levels of anti-wild type *H*. *pylori* antibodies in mice immunized with urease-expressing *S*. *typhimurium* construct A were -20-fold higher at three weeks and 34-fold higher ten weeks after immunization with respect to the pre-immune serum. The serum IgA antibody response against the urease-deficient *H. pylori* strain at 3 and 10 weeks was low in all groups of mice including the mice immunized with *Salmonella* construct A (Fig. 5, panel B), indicating that most of the IgA antibody response in immunized mice is directed against the urease subunits. Low serum antibody responses against wild type *H. pylori* were also observed at ten weeks in non-immunized mice suggesting that the *H. pylori* challenge given three weeks earlier was enough to induce a specific antibody response in these mice. The IgA response to wild type *H. pylori* in mice immunized for three weeks with *S*. *typhimurium* SL3261 (*Salmonella* control group) increased moderately, which may be explained by the presence of antigens in *Salmonella* that are able to induce cross-reacting antibodies against *H. pylori.* In contrast, the antibody response against the urease-deficient *H. pylori* strain in immunized mice was as low as the antibody response of non-immunized mice (Fig 5, panel B). This result suggests that most of the antibody response observed in immunized mice was against urease. Low antibody response against the urease-negative mutant was observed in the 10 weeks sera from mice given PBS or immunized with *S*. *typhimurium* SL3261, suggesting that the antibody response observed is due to the specific immune response against the *H. pylori* antigens given to these mice three weeks earlier during challenge. A low antibody response against the urease-deficient *H. pylori* strain was observed at three weeks in mice immunized with *Salmonella* either expressing or not expressing urease, but was absent in the mice given PBS. This confirms the presence of cross-reacting epitopes between proteins from *Salmonella* and H. *pylori,* respectively. (Construct B): The serological analysis of mice immunized with the construct B series achieved similar results indicating that higher production of antigen by recombinant *Salmonella* does not significantly increase antibody response.

### Analysis of anti-urease antibodies in mouse sera by immunoblotting.

Expression of UreA and UreB from *S*. *typhimurium* necessary for the induction of mice specific immune response against *H*. *pylori* was analyzed. Identification of *in vivo* expression of UreA and UreB was carried out by looking for anti-UreA and anti-UreB antibodies in serum of mice immunized with *Salmonella* construct A and control mice. *H. pylori* whole-cell antigens were prepared from the wild type *H. pylori* strain P1. Bacteria were recovered from 3 serum plates, resuspended in PBS, and harvested by 10 min centrifugation at 5,000 g. The cell pellet was resuspended in 10mM Tris-HCl and 10 mM EDTA, (pH 8.0) and cell-density adjusted to standard A₅₉₀=1.0 in all probes. The bacterial suspension was mixed with same volume of SDS-sample buffer (Sambrook, 1989) and boiled for 5 min. 20 µl Pellet were loaded onto a SDS-10% polyacrylamide gel. The proteins were electro-blotted onto a nitrocellulose membrane and cut into strips which were blocked for 45 min at room temperature in 10 (v/w)% non-fat milk Tris-buffer-saline (TBS) (TrisHCl 100mM, NaCl 150mM, pH 7.2). After three washes in TBS-0,05 (v/v)% Tween-20, a 1:80 dilution of mouse serum in 5 (w/v)% non-fat milk-TBS was added to the strips and incubated overnight at 4°C. Sera was obtained from mice non-immunized and immunized with *Salmonella.* After three washes, the strips were incubated with a goat anti-mouse IgG horse-radish peroxidase conjugate (Sigma) diluted 1:3000 in 5 (w/v)% non-fat milk-TBS. The ECL chemi-luminescence detection kit (Amersham, Germany) was used for development of blots according to the supplier' s directions.

Serum from immunized and non-immunized mice was obtained 3 weeks after immunization prior to the challenge with *H*. *pylori* and tested against whole-cell lysates of the wild type *H. pylori* P1 strain expressing UreA and UreB. Proteins of 67 kDa and 30 kDa in size, corresponding to UreB and UreA, respectively, were recognized by serum from immunized mice immunized with construct A. These bands were not observed in strips tested with serum from non-immunized mice or mice immunized with *Salmonella* only, suggesting that urease expressed by the *Salmonella* vaccine strain was able to induce a specific antibody response against both UreA and UreB of a wild type *H. pylori* strain. Similar results were obtained with construct B.

### Example 6

### Determination of H. pylori colonisation in mice pretreated with the various recombinant S. typhimurium strains expressing ureA/ureB subunits in H. pylori mouse model

### Processing of stomach and measurement of urease activity.

Urease-test: Analysis of protection against stomach colonization by *H*. *pylori* was performed by testing for urease activity in the antral portion of the mouse stomach. Measurement of urease activity is a very reliable, sensitive and specific method to test for the presence of *H. pylori* infection (NIH consensus development on *Helicobacter pylori* in peptic ulcer disease. 1994. Helicobacter pylori in peptic ulcer disease. JAMA. 272:65) and is routinely used in clinical settings (Kawanishi, M., S. et al 1995. J. Gastroenterol. 30:16-20; Kamija, S. et al 1993. Eur. J. Epidemiol. 9:450-452; Conti-Nibali, S. et al 1990. Am. J. Gastroenterol. 85:1573-1575) and in animal research (Gottfried, M. R. et al 1990. Am. J. Gastroenterol. 85:813-818). The Jatrox-test (Röhn-Pharma GmbH, Weiterstadt, Germany) was used according to the suppliers directions. Eleven weeks after immunization with *Salmonella,* mice were sacrificed and the stomach was carefully removed under aseptic conditions. The stomach was placed in ice-cold PBS in an sterile container, and the mucosa was exposed by making an incision along the minor curvature with a sterile blade. The stomach was rinsed with PBS to remove food residues and dissected to isolate the antral region from the corpus region. Half of the antral portion of the stomach was immediately placed inside an Eppendorf tube containing 500 µl of the urease substrate from Jatrox-test. The stomach sample was incubated 4 h at room temperature and the absorbance at 550 nm (A₅₅₀) measured. The urease activity values obtained from the stomach of naive mice, which did not undergo immunization or challenge, were used to determine the baseline. The baseline corresponded to the average urease activity value from five naive mice stomachs tested plus two times the standard deviation of this average. Urease activity values higher than the baseline were considered *H. pylori* colonization positive and values below the baseline were considered *H. pylori* colonization negative.

**Cultivation experiment:** The left portion of the antral region of stomachs obtained from mice challenged with the streptomycin resistant *H. pylori* strain P76 were plated on serum plates supplemented with 200 µg/ml of streptomycin and incubated under standard conditions. After three days incubation, bacteria were identified as *H. pylori* based on colony morphology, microscopic examination, and urease activity. The number of colony forming units (CFU) of *H. pylori* grown on plates was determined from each mouse stomach sample.

**Urease test** (**Construct A vs. B**): Mice immunized with ~5.0X10⁹ CFU of *Salmonella* and challenged with 1.0X10⁹ CFU of *H. pylori* strain P49, as well as control mice, were sacrificed under anesthesia and a section of the antral region of the stomach was taken for measurement of urease activity. As shown in Fig. 6, 100% of the mice immunized with UreA and B delivered by *Salmonella* construct A had urease activity below the baseline, indicating the absence of *H*. *pylori* colonisation. In contrast, 100 % of the non-immunized mice (PBS) and the mice immunized with *S. typhimurium* strain SL3261 alone, had urease activity measurements far above the baseline indicating stomach colonization by *H. pylori.* The naive group of mice, which did not undergo immunization or challenge, was used to set the baseline of urease activity.

*Salmonella* of the construct B-series had urease activity values above the baseline indicating stomach colonization by *H. pylori* challenge strain. However, the urease activities within this group were lower as in the controls suggesting a partial protection status of mice immunized with the Salmonella construct B series (Figure 6). Both Salmonella constructs, A and B, mediate similar antibody response but differed in expression of ureA and ureB. We conclude from this that the quantity of expressed urease antigen is relevant to gain optimal protection.

**Construct A**: To correlate stomach colonization by *H*. *pylori* with urease activity a new protection experiment was performed by immunizing mice orally with *Salmonella* construct A and challenging them with the streptomycin resistant *H*. *pylori* P76 strain. Urease activity values correlated with the number of CFU of *H. pylori* identified. In two of the five mice immunized with urease-expressing *Salmonella,* no *H. pylori* CFU were detected and the average number of CFU in all five immunized mice was only 62. In contrast, the number of CFU in non-immunized mice was 2,737, which corresponds to 44-fold higher colonization. These data indicate that mice immunized with urease-expressing *Salmonella* were able to eliminate or significantly decrease colonizing *H. pylori* from mouse stomachs.

## Claims

1. Pharmaceutical composition comprising as an active agent an immunologically protective living vaccine which is a recombinant attenuated *Salmonella* cell which comprises at least one heterologous nucleic acid molecule encoding a *Helicobacter* antigen wherein said *Salmonella* cell is capable of expressing said nucleic acid molecule or capable of causing the expression of said nucleic acid molecule in a target cell, and wherein the *Helicobacter* antigen is urease, a urease subunit or an immunologically reactive fragment thereof.

2. The composition according to claim 1, wherein the *Salmonella* cell is a *Salmonella* aro mutant cell.

3. The composition according to claims 1 or 2, wherein said nucleic acid molecule encoding a *Helicobacter* antigen is capable of being expressed phase variably.

4. The composition according to claim 3, wherein said nucleic acid molecule encoding a *Helicobacter* antigen is under the control of an expression signal which is substantially inactive in the *Salmonella* cell and which is capable of being activated by a nucleic acid reorganization caused by a nucleic acid reorganization mechanism in the *Salmonella* cell.

5. The composition according to claim 4, wherein the expression signal is a bacteriophage promoter and the activation is caused by a DNA reorganization resulting in the production of a corresponding bacteriophage RNA polymerase in the *Salmonella* cell.

6. The composition according to any one of claims 1 to 5, wherein said *Salmonella* cell further comprises at least one second nucleic acid molecule encoding an immunomodulatory polypeptide, wherein said *Salmonella* cell is capable of expressing said second nucleic acid molecule.

7. The composition according to any one of claims 1 to 6, together with pharmaceutically acceptable diluents, carriers and adjuvants.

8. The composition according to claim 7 which is suitable for administration to a mucosal surface or via the parenteral route.

9. A method for the preparation of a living vaccine comprising formulating a pharmaceutical composition according to any one of claims 1 to 6 in a pharmaceutically effective amount with pharmaceutically acceptable diluents, carriers and/or adjuvants.

10. The method of claim 9, including the preparation of a recombinant attenuated *Salmonella* cell comprising the steps:
(a) inserting a nucleic acid molecule encoding a *Helicobacter* antigen into an attenuated *Salmonella* cell, wherein a recombinant attenuated *Salmonella* cell is obtained, which is capable of expressing said nucleic acid molecule or is capable of causing the expression of said nucleic acid molecule in a target cell, and wherein the *Helicobacter* antigen is urease, a urease subunit or an immunologically reactive fragment thereof, and
(b) cultivating said recombinant attenuated *Salmonella* cell under suitable conditions.

11. The method according to claim 10, wherein said nucleic acid molecule encoding a *Helicobacter* antigen is located on an extrachromosomal plasmid or inserted in the chromosome.

## Patentansprüche

1. Pharmazeutische Zusammensetzung, welche als Wirkstoff eine immunologisch protektive Lebendvakzine umfasst, welche eine rekombinante attenuierte *Salmonella*-Zelle ist, welche wenigstens ein heterologes Nukleinsäuremolekül umfasst, welches ein *Helicobacter*-Antigen kodiert, wobei die *Salmonella*-Zelle das Nukleinsäuremolekül exprimieren kann oder die Expression des Nukleinsäuremoleküls in einer Zielzelle verursachen kann und wobei das *Helicobacter*-Antigen Urease, eine Urease-Untereinheit oder ein immunologisch reaktives Fragment davon ist.

2. Zusammensetzung gemäß Anspruch 1, wobei die *Salmonella*-Zelle eine aromutierte *Salmonella*-Zelle ist.

3. Zusammensetzung gemäß Anspruch 1 oder 2, wobei das Nukleinsäuremolekül, welches ein *Helicobacter*-Antigen kodiert, phasenvariabel exprimiert werden kann.

4. Zusammensetzung gemäß Anspruch 3, wobei das Nukleinsäuremolekül, welches ein *Helicobacter*-Antigen kodiert, unter der Kontrolle eines Expressionssignals steht, welches in der *Salmonella-Zelle* im Wesentlichen inaktiv ist und welches durch eine Nukleinsäure-Reorganisation aktiviert werden kann, welche durch einen Nukleinsäure-Reorganisationsmechanismus in der *Salmonella*-Zelle verursacht wird.

5. Zusammensetzung gemäß Anspruch 4, wobei das Expressionssignal ein Bakteriophagenpromotor ist und die Aktivierung durch eine DNA-Reorganisation verursacht wird, welche zur Produktion einer korrespondierenden Bakteriophagen-RNA-Polymerase in der *Salmonella*-Zelle führt.

6. Zusammensetzung gemäß einem der Ansprüche 1 bis 5, wobei die *Salmonella*-Zelle weiterhin wenigstens ein zweites Nukleinsäuremolekül umfasst, welches ein immunmodulatorisches Polypeptid kodiert, wobei die *Salmonella*-Zelle das zweite Nukleinsäuremolekül exprimieren kann.

7. Zusammensetzung gemäß einem der Ansprüche 1 bis 6, zusammen mit pharmazeutisch akzeptablen Verdünnern, Trägern und Hilfsstoffen.

8. Zusammensetzung gemäß Anspruch 7, welche zur Verabreichung an einer mukosalen Oberfläche oder über den parenteralen Weg geeignet ist.

9. Verfahren zur Herstellung einer Lebendvakzine umfassend Formulieren einer pharmazeutischen Zusammensetzung gemäß einem der Ansprüche 1 bis 6 in einer pharmazeutisch wirksamen Menge mit pharmazeutisch akzeptablen Verdünnern, Trägern und/oder Hilfsstoffen.

10. Verfahren gemäß Anspruch 9, einschließend die Herstellung einer rekombinanten attenuierten *Salmonella*-Zelle, umfassend die Schritte:
(a) Inserieren eines Nukleinsäuremoleküls, welches ein *Helicobacter-*Antigen kodiert, in eine attenuierte *Salmonella*-Zelle, wobei eine rekombinante attenuierte *Salmonella*-Zelle erhalten wird, welche das Nukleinsäuremolekül exprimieren kann oder die Expression des Nukleinsäuremoleküls in einer Zielzelle verursachen kann und wobei das *Helicobacter*-Antigen Urease, eine Urease-Untereinheit oder ein immunologisch reaktives Fragment davon ist, und
(b) Kultivieren der rekombinanten attenuierten *Salmonella*-Zelle unter geeigneten Bedingungen.

11. Verfahren gemäß Anspruch 10, wobei das Nukleinsäuremolekül, welches ein *Helicobacter-*Antigen kodiert, auf einem extrachromosomalen Plasmid angeordnet ist oder in das Chromosom inseriert wird.

## Revendications

1. Composition pharmaceutique comprenant, en tant qu'agent actif, un vaccin vivant protecteur immunologiquement, qui est une cellule de *Salmonella* recombinante atténuée et qui comprend au moins une molécule d'acide nucléique hétérologue codant pour un antigène d'*Helicobacter,* dans laquelle ladite cellule de *Salmonella* est capable d'exprimer ladite molécule d'acide nucléique ou capable de provoquer l'expression de ladite molécule d'acide nucléique dans une cellule cible, et dans laquelle l'antigène d'*Helicobacter* est l'uréase, une sous-unité uréase ou un de ses fragments réactifs immunologiquement.

2. Composition selon la revendication 1, dans laquelle la cellule de *Salmonella* est une cellule de *Salmonella* aro mutante.

3. Composition selon les revendications 1 ou 2, dans laquelle la molécule de l'acide nucléique codant pour un antigène *Helicobacter* est capable d'être exprimée de manière variable en fonction de la phase.

4. Composition selon la revendication 3, dans laquelle ladite molécule d'acide nucléique codant pour un antigène d'*Helicobacter* est sous le contrôle d'un signal d'expression qui est sensiblement inactif dans la cellule de *Salmonella* et qui est capable d'être activé par une réorganisation d'acide nucléique provoquée par un mécanisme de réorganisation d'acide nucléique dans la cellule de *Salmonella.*

5. Composition selon la revendication 4, dans laquelle le signal d'expression est un promoteur bactériophage et l'activation est provoquée par une réorganisation d'ADN se traduisant par la production d'une polymérase ARN bactériophage correspondante dans la cellule de *Salmonella.*

6. Composition selon l'une quelconque des revendications 1 à 5, dans laquelle ladite cellule de *Salmonella* comprend de plus au moins une seconde molécule d'acide nucléique codant pour un polypeptide immunomodulateur, dans laquelle ladite cellule de *Salmonella* est capable d'exprimer ladite seconde molécule d'acide nucléique.

7. Composition selon l'une quelconque des revendications 1 à 6, conjointement avec des diluants, supports et adjuvants pharmaceutiquement acceptables.

8. Composition selon la revendication 7 qui est appropriée pour l'administration sur une surface de muqueuse ou via la voie parentérale.

9. Procédé pour la préparation d'un vaccin vivant comprenant la formulation d'une composition pharmaceutique selon l'une quelconque des revendications 1 à 6, dans une quantité pharmaceutiquement efficace, avec des diluants, supports et/ou adjuvants pharmaceutiquement acceptables.

10. Procédé selon la revendication 9, comprenant la préparation d'une cellule de *Salmonella* recombinante atténuée et comprenant les étapes :
(a) introduire une molécule d'acide nucléique codant pour un antigène d'*Helicobacter* dans une cellule de *Salmonella* atténuée, dans laquelle une cellule de *Salmonella* atténuée recombinante est obtenue, qui est capable d'exprimer ladite molécule d'acide nucléique ou qui est capable de provoquer l'expression de ladite molécule d'acide nucléique dans une cellule cible, et dans laquelle l'antigène d'*Helicobacter* est l'uréase, une sous-unité uréase ou un de ses fragments réactifs immunologiquement, et
(b) mettre en culture ladite cellule de *Salmonella* atténuée recombinante dans des conditions appropriées.

11. Procédé selon la revendication 10, dans lequel ladite molécule d'acide nucléique codant pour un antigène d'*Helicobacter* est située sur un plasmide extrachromosomique ou insérée dans le chromosome.


### SEQUENCE LISTING

(1) GENERAL INFORMATION:
   (i) APPLICANT:
      (A) NAME: Max-Planck-Gesellechaft zur Foerderung der Wissenschaften e.V. Berlin
      (B) STREET: Hofgartenstr. 2
      (C) CITY: Muenchen
      (E) COUNTRY: Germany
      (F) POSTAL CODE (ZIP): 80539
   (ii) TITLE OF INVENTION: Helicobacter pylori live vaccine
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 5:
   (i) SEQUENCE CHARACTERISTICS:
      (A) LENGTH: 656 base pairs
      (B) TYPE: nucleic acid
      (C) STRANDEDNESS: double
      (D) TOPOLOGY: both
   (ix) FEATURE:
      (A) NAME/KEY: CDS
      (B) LOCATION:567..656
   (xi) SEQUENCE DESCRIPTION: SEQ ID NO: 5:
   AGATCTATGA ATCTATGATA TCAACACTCT TTTTGATAAA TTTTCTCGAG GTACCGAGCT 60
   TGAGGCATCA AATAAAACGA AAGGCTCAGT CGAAAGACTG GGCCTTTCGT TTTATCTGTT 120
   GTTTGTCGGT GAACGCTCTC CTGAGTAGGA CAAATCCGCC GGGAGCGGAT TTGAACGTTG 180
   CGAAGCAACG GCCCGGAGGG TGGCGGGCAG GACGCCCGCC ATAAACTGCC ACAAGCTCGG 240
   TACCGTTGAT CTTCCTATGG TGCACTCTCA GTACAATCTG CTCTGATGCG CTACGTGACT 300
   GGGTCATGGC TGCGCCCCGA CACCCGCCAA CACCCGCTGA CGCGCCCTGA CGGGCTTGTC
      360
   TGCTCCCGGC ATCCGCTTAC AGACAAGCTG TGACCGTCTC CGGGAGCTGC ATGTGTCAGA
      420
   GGTTTTCACC GTCATCACCG AAACGCGCGA GGCCCAGCGC TTCGAACTTC TGATAGACTT
      480
   CGAAATTAAT ACGACTCACT ATAGGGAGAC CACAACGGTT TCCCTCTAGA AATAATTTTG
      540
   TTTAACTTTA AGAAGGAGAT ATACAT ATG AAA CTG ACT CCC AAA GAG TTA GAC
      593
                                Met Lys Leu Thr Pro Lys Glu Leu Asp
                                    520                 525
   AAG TTG ATG CTC CAC TAC GCT GGA GAA TTG GCT AAA AAA CGC AAA GAA
      641
   Lys Leu Met Leu His Tyr Ala Gly Glu Leu Ala Lys Lys Arg Lys Glu
           530                 535                 540
   AAA GGC ATT AAG CTT
      656
   Lys Gly Ile Lys Leu
       545
(2) INFORMATION FOR SEQ ID NO: 6:
   (i) SEQUENCE CHARACTERISTICS:
      (A) LENGTH: 30 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Lys Leu Thr Pro Lys Glu Leu Asp Lys Leu Met Leu His Tyr Ala
    1               5                   10                  15
   Gly Glu Leu Ala Lys Lys Arg Lys Glu Lys Gly Ile Lys Leu
               20                  25                  30